# EUROPEAN PATENT APPLICATION

(11) **EP 0 761 252 A1**
(43) Date of publication of application: **12.03.1997**
(21) Application number: 96114238.7
(22) Date of filing: 05.09.1996
(51) Int. Cl.: A61M 29/02

(54) **A perfusion catheter**

(30) Priority: 06.09.1995 JP 229399/95
(71) Applicant: Kabushiki Kaisha Vayu, Nagoya-shi, Aichi-ken (JP)
(72) Inventor: Hirayama, Haruo, c/o K.K. Vayu, Nagoya-shi, Aichi-ken (JP); Tsutsui, Nobumasa, c/o K.K. Vayu, Nagoya-shi, Aichi-ken (JP)
(74) Representative: Tiedtke, Harro, Dipl.-Ing.

(57) **Abstract**

A perfusion catheter having an improved fluidity of blood in a blood passage and an improved operability in a blood vessel. The perfusion catheter is provided with an outer tube inserted through a balloon and an inner tube having a tapered or pointed tip disposed in the outer tube. The tapered tip of the inner tube is projected out of a forward open end of the outer tube and can retreat inside the forward open end. When the inner tube is moved back behind bores formed in a side wall of the outer tube, the bores communicate via a second lumen to the forward open end, thereby passing blood. The outer periphery inserted through the balloon of the outer tube is securely wound around with a coil.

## Description

### FIELD OF THE INVENTION

This invention relates to a perfusion catheter that expands the stenosed portion of a blood vessel while accommodating blood flow in the blood vessel.

### BACKGROUND OF THE INVENTION

Conventionally, as a variety of a balloon catheter for use during the percutaneous transluminal coronary angioplasty (referred to as PTCA hereinafter) or other operation, a perfusion catheter provided with a blood passage connecting the forward end and the rear end of a balloon is known. Blood can flow through the blood passage between the forward end and the rear end of the balloon, even when the balloon is swelled.

In the perfusion catheter, the balloon can be expanded, while passing blood downstream in the blood vessel. Therefore, the perfusion catheter, rather than a normal balloon catheter, is used when a patient is sensitive to ischemia.

If after PTCA, a portion of blood vessel wall separates from the blood vessel and the blood vessel is obstructed or is likely to be obstructed, then a lesion part of the blood vessel must be expanded over a long period of time so that the damaged part of the blood vessel wall can heal. This is another case where the use of a perfusion catheter is preferred.

It is desirable to enlarge the blood passage as much as possible to facilitate smooth and effective blood flow.

It is also desirable that the forward end of the balloon be as thin as possible so that the perfusion catheter can be easily introduced in the blood vessel and the balloon can be easily introduced to the stenosed portion of the blood vessel.

The obvious shortcoming of conventional perfusion catheter is that when the blood passage is enlarged, the forward end of the balloon is also enlarged. Contrarily, when the forward end of the balloon is narrowed, the blood passage necessarily narrows. Therefore, the adjustment of the thickness of the blood passage, alone, cannot improve the blood flow in the blood passage, and at the same time, improve the guiding operation of the perfusion catheter into the blood vessel.

### SUMMARY OF THE INVENTION

Wherefore, an object of the present invention is to provide a perfusion catheter having improved blood flow in the blood passage and improved operability in the blood vessel.

To achieve this or other object, the present invention provides a perfusion catheter provided with a catheter shaft having a lumen for feeding or discharging fluid, a balloon provided at the forward end of the catheter shaft for swelling or contracting by means of the fluid fed or discharged via the lumen, and a blood passage connecting the forward end of the balloon to the rear end thereof for passing blood between the forward end and the rear end, even when the balloon is swelled. The perfusion catheter is further composed of a movable tip member able to project out of a forward open end of the blood passage. The rear end of the movable tip member is almost the same in size as the forward open end of the blood passage. The movable tip member is tapered from its rear end toward its forward end, and can be retracted inside the forward open end of the blood passage, such that blood can flow without being obstructed in the blood passage.

The perfusion catheter is provided with an outer cylindrical member disposed along the axis of the balloon and an inner cylindrical member slidably inserted through the outer cylindrical member. The blood passage is formed by bores formed in the side wall of the outer cylindrical member, behind the balloon, communicating via a lumen in the outer cylindrical member to the forward open end of the outer cylindrical member. The movable tip member is formed by the tip of the inner cylindrical member tapered from its rear end toward its forward end.

In the perfusion catheter, a metallic wire is helically secured around a part of the outer periphery of the outer cylindrical member through the balloon.

The movable tip member projected out of the forward open end of the blood passage has the rear end almost the same in size as the forward open end of the blood passage, and has the forward end tapered or pointed from the rear end thereof. In this way, the blood passage can be enlarged, without adversely affecting the operability of the perfusion catheter within the blood vessel. Although the forward open end of the blood passage is enlarged, the tip of the movable tip member tapers from the forward open end. Therefore, the perfusion catheter can smoothly advance in the blood vessel. Since the movable tip member is easily guided into the stenosed portion of the blood vessel, the balloon following the movable tip member can also be easily guided into the stenosed portion.

After the balloon reaches the desired retention position, the movable tip member can be retreated back into the forward open end of the blood passage, such that blood can flow without being obstructed through the blood passage. As aforementioned, by providing the movable tip member, a relatively large blood passage can be realized. Therefore, blood flows more smoothly and at an increased rate, as compared to conventional perfusion catheters.

According to the invention, the perfusion catheter has an enlarged blood passage for facilitating blood flow, and a movable tip member for easily guiding the perfusion catheter in the blood vessel toward the lesion portion.

The tapered configuration of the movable tip member is not limited, as long as the tip of the movable tip member is thinner than the rear end thereof. Specifically, the tapered configuration includes a longitudinal portion having the same thickness, a partially narrowed portion and a partially enlarged portion.

When retracted, a portion of the movable tip member can be left inside the blood passage, so long as blood flows in the blood passage. Preferably, the movable tip member is retreated completely outside the blood passage so that blood flow is not obstructed. When the movable tip member or the blood passage is configured or sized such that a sufficient clearance is obtained between the movable tip member and the wall of the blood passage, a portion of the movable tip member can be left in the blood passage, so long as blood flows in the blood passage.

For example, to form the movable tip member, the tip of the inner cylindrical member of the perfusion catheter is tapered or pointed. Even if the outer cylindrical member is thickened and the forward open end of the outer cylindrical member is enlarged, the tip of the inner cylindrical member can be thinner than the forward open end of the outer cylindrical member. When the perfusion catheter with the tip of the inner cylindrical member projected from the forward open end is inserted into the blood vessel, the perfusion catheter can smoothly advance in the blood vessel. The tip of the inner cylindrical member can easily reach the stenosed portion. The balloon can be also easily introduced into the stenosed portion following the tip of the inner cylindrical member.

Since the inner cylindrical member is slidably inserted through the outer cylindrical member, it can be moved to the rear part behind the bores formed in the side wall of the outer cylindrical member. Therefore, after the balloon reaches the desired retention position, by retreating the inner cylindrical member, blood can flow via the lumen in the outer cylindrical member.

Furthermore, a guide wire can be inserted through the lumen in the inner cylindrical member. The guide wire is positioned in the blood vessel beforehand, so that the tip of the inner cylindrical member is guided by the guide wire and smoothly advances in the blood vessel. This configuration is known as an over the wire type of the perfusion catheter.

Alternatively, a guide wire with a tapered or pointed member secured to the tip thereof can be used. This configuration forms a so-called on the wire type of the perfusion catheter. The perfusion catheter is moved forward in the blood vessel together with the guide wire. After the balloon reaches the retention position, the tapered member is retreated via the guide wire.

Threads can be provided in the outer periphery of the inner cylindrical member and in the inner periphery of the outer cylindrical member. By rotating or screwing the inner cylindrical member in the outer cylindrical member, the inner cylindrical member can be retreated. The engaged threads prevent the inner cylindrical member from moving freely within the outer cylindrical member, even when the perfusion catheter is moved forward in the blood vessel. The advance and retreat of the inner cylindrical member can be finely controlled by rotating the inner cylindrical member. Instead of providing engageable threads, coils can be densely wound around the peripheries of the inner and/or outer cylindrical members.

When a second lumen is formed parallel to the first lumen for supplying and discharging fluid in the catheter shaft, the catheter shaft can serve as the aforementioned outer cylindrical member. Alternatively, the outer cylindrical member can be disposed in the perfusion catheter separately from the catheter shaft. Specifically, separately from the catheter shaft, the outer cylindrical member slightly longer than the balloon is disposed through the balloon and blood can flow between both open ends of the outer cylindrical member. The movable tip member is provided such that it can pulled from the rear open end of the outer cylindrical member. The pulled movable tip member is retained in the side face of the catheter shaft.

The part of the outer cylindrical member passing through the balloon forms the blood passage inside. Even when pressure is applied from the swelled balloon to the outer cylindrical member, the lumen forming the blood passage must be prevented from being obstructed. For the purpose the outer cylindrical member is formed of highly rigid material.

The metallic wire secured on the outer cylindrical member loses no flexibility even when the outer cylindrical member is bent. When the balloon is swelled to apply high pressure, the lumen in the outer cylindrical member is protected by the metallic wire and blood can flow stably in the blood passage.

### BRIEF DESCRIPTION OF THE DRAWINGS

The invention will now be described, by way of example, with reference to the drawings, in which:
Fig. 1A is a side view of a perfusion catheter made in accordance with the present invention.
Fig. 1B is a side view of an outer catheter of the perfusion catheter.
Fig. 1C is a side view of an inner catheter of the perfusion catheter.
Fig. 2A is a sectional view of the tip of the perfusion catheter showing the initial position of the inner catheter.
Fig. 2B is a sectional view showing the retreated position of the inner catheter.
Fig. 3 is a view showing the expansion of a stent by means of the perfusion catheter.
Fig. 4 is a side view of a second embodiment of a perfusion catheter made in accordance with the present invention.

### DETAILED DESCRIPTION OF THE PREFERRED EMBODIMENTS

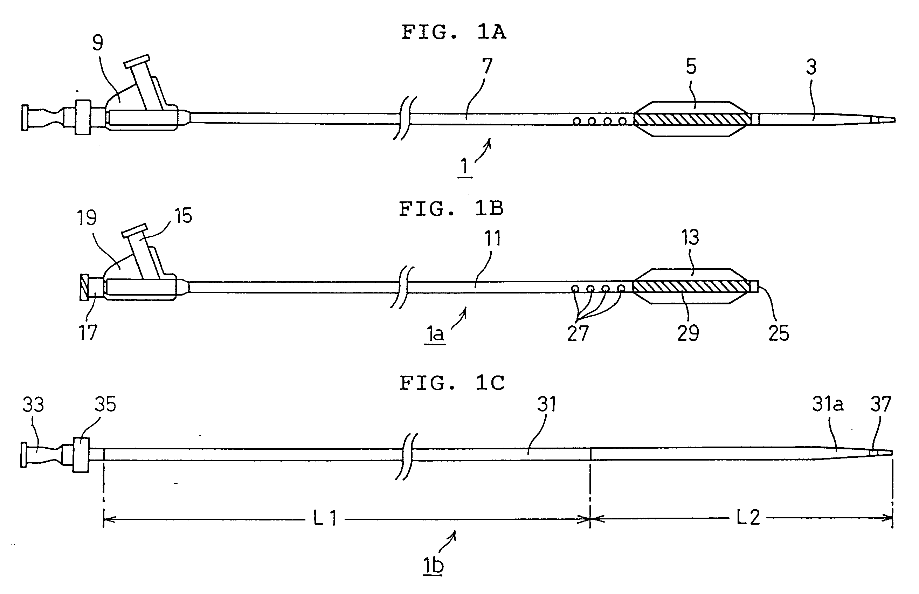

In the first embodiment, as shown in Fig. 1A, a perfusion catheter 1 is composed of a tip 3, a balloon part 5, a catheter shaft 7 and a connector part 9. The perfusion catheter 1 has an inner catheter 1b (shown in Fig. 1C) disposed inside an outer catheter 1a (shown in Fig. 1B).

As shown in Fig. 1B the outer catheter 1a is provided with an outer tube 11, a balloon 13 disposed at the tip of the outer tube 11, and a connector 19 provided at the rear end of the outer tube 11 having a balloon expansion port 15 and an inner catheter operation port 17.

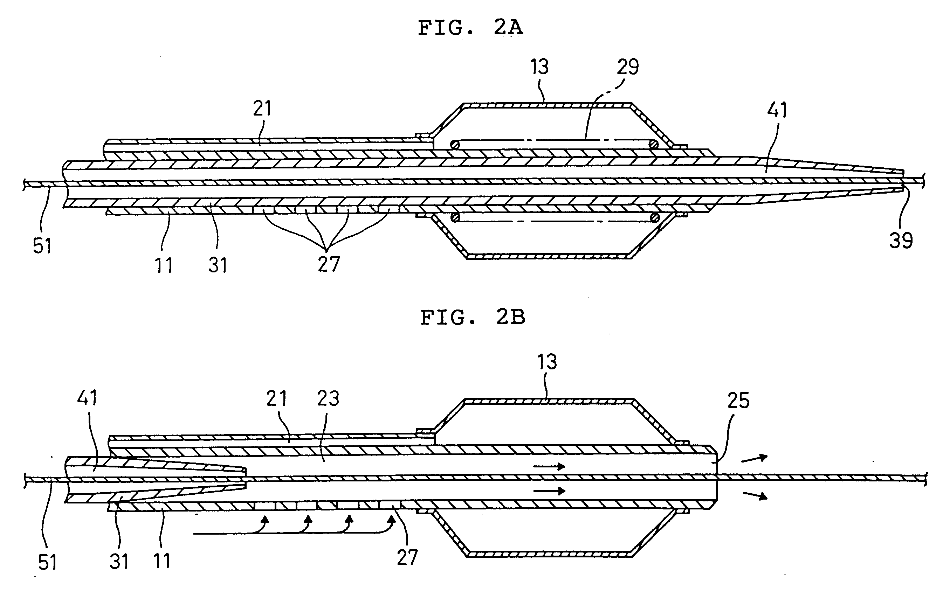

The outer tube 11 is passed through the balloon 13 adhered to the outer periphery of outer tube 11. As shown in Fig. 2B, first and second lumens 21 and 23 are provided parallel with each other inside the outer tube 11. The forward end of the first lumen 21 communicates with the inside of balloon 13 and the rear end of first lumen 21 communicates with the balloon expansion port 15 shown in Fig. 1B. Liquid is supplied via the balloon expansion port 15 through the first lumen 21 into the balloon 13. The pressure of the liquid causes the balloon 13 to swell. As shown in Fig. 2B, the second lumen 23 leads to a forward open end 25 thereof and leads to the inner catheter operation port 17 shown in Fig. 1B at the rear end thereof. As shown in Fig. 2A, the inner catheter 1b (shown in Fig. 1C) is slidably inserted from the rear end of second lumen 23. The outer tube 11 has side bores 27 for communicating with the second lumen 23 behind the balloon 13 in the side wall thereof. A stainless coil 29 is secured around the outer periphery of the part of outer tube 11 passed through the balloon 13.

As shown in Fig. 1C, the inner catheter 1b is composed of an inner tube 31 having a tapered tip 31a, a connector 35 having a guide wire insertion port 33 and a metal marker 37 for use when the position of tapered tip 31a is confirmed with X-radiation.

The inner tube 31 is entirely flexible. By adjusting the material and thickness of the inner tube 31, the part extended along length L2, shown in Fig. 1C, is more flexible than the part extended along length L1. The part extended along length L1 of the inner tube 31 easily transmits an extrusion force applied to the proximal end of the perfusion catheter 1 for advancing the perfusion catheter 1 in the blood vessel to the distal tip of the perfusion catheter 1. As shown in Fig. 2A, the inner tube 31 has a lumen 41 which can accommodate a guide wire 51 extended from a forward open end 39 thereof to the guide wire insertion port 33 (shown in Fig. 1C).

As shown in Fig. 1A, the tip 3 of the perfusion catheter 1 is formed of the tapered tip 31a of inner tube 31 projected from the forward open end 25 of outer tube 11. The largest outer diameter of the tapered tip 31a is about the same in size as the inner diameter of the forward open end 25 of the outer tube 11. Therefore, the tapered tip 31a is projected from the outer tube 11, there is no step between the inner tube 31 and the outer tube 11, which facilitates the insertion of the perfusion catheter 1 into the blood vessel. The tapered tip 31a of inner tube 31 can be retracted inside the forward open end 25 of the outer tube 11. As shown in Fig. 2B, the tapered tip 31a can be retracted behind the side bores 27. By retracting the inner tube 31 in this manner, the side bores 27 can communicate via the second lumen 23 to the forward open end 25, thereby facilitating blood flow as shown by arrows in Fig. 2B.

In operation of the perfusion catheter 1, the pressure inside the balloon 13 is reduced via the balloon expansion port 15 shown in Fig. 1B, thereby contracting the balloon 13 beforehand. A guide catheter, not-shown, is inserted in a blood vessel until the tip of the guide catheter is just before the lesion portion of the blood vessel to be expanded. Subsequently, the perfusion catheter 1, with the guide wire 51 passed therethrough, is inserted in the guide catheter. After the guide wire 51 is advanced and passed through the lesion portion, the perfusion catheter 1 is advanced along the guide wire 51.

Since the tip 3 of perfusion catheter 1 is tapered, the perfusion catheter 1 can be easily advanced even in the blood vessel and can be easily inserted even in a stenosed lesion portion of the blood vessel.

After the balloon 13 reaches the lesion portion, liquid is fed via the balloon expansion port 15, thereby swelling the balloon 13 and expanding the lesion portion. After the balloon 13 is swelled, the inner tube 31 is retracted from the position shown in Fig. 2A to the position shown in Fig. 2B, such that the side bores 27 communicate via the second lumen 23 to the forward open end 25. Therefore, blood flows, as shown by the arrows in Fig. 2B, toward the downstream in the blood vessel.

Preferably, the diameters of the forward open end 25 and second lumen 23 in the perfusion catheter 1 are rather large, to accommodate a large volume of blood.

In general, a large forward open end 25 makes it difficult to guide the perfusion catheter 1 in the blood vessel or insert the perfusion catheter 1 to the stenosed portion of the blood vessel. However, the tapered or pointed tip 31a of the inner tube 31 projected from the outer tube 11, as shown in Fig. 2A, allows the perfusion catheter 1 to be easily guided in the blood vessel and inserted into the stenosed portion of the blood vessel. Furthermore, since the inner tube 31 is not required to facilitate blood flow, the tapered point 31a can be thinner than the tip of the usual perfusion catheter. The perfusion catheter 1 can be easily and effectively guided in the blood vessel and introduced into the stenosed portion of the blood vessel.

When the inner tube 31 is retreated, the stainless coil 29 secured around the outer periphery of the outer tube 11 prevents the outer tube 11 from being deformed by the inner pressure of the balloon 13, thus ensuring that the second lumen 23 remains unobstructed.

The perfusion catheter 1 can directly expand the blood vessel as aforementioned, and can also be used for delivering a stent, for example.

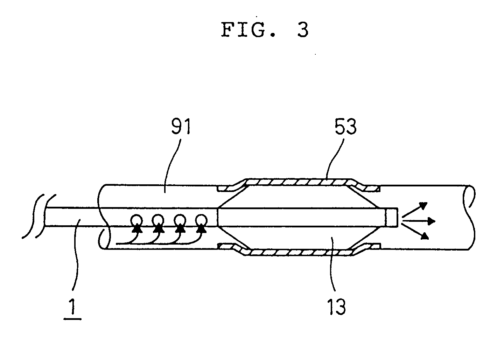

As shown in Fig. 3, a stent 53, a metal tube having a netted side wall, is attached around the outer periphery of the balloon 13 for being conveyed in a blood vessel 91. By swelling the balloon 13, the meshes in the netted side wall of the stent 53 are expanded and the inner diameter of the stent 53 is enlarged, thereby expanding the blood vessel 91.

Usually, the stent is preferably expanded rather excessively, for example, 100% to 120% relative to the original inner diameter of the blood vessel. Expanding the lumen of the stent in a substantially cylindrical fashion decreases the postoperative stenosis recurrence rate. Therefore, the stent is required to be expanded under high pressure over a long time period.

For the perfusion catheter 1 of the first embodiment, by retreating the forward end thereof, blood can effectively flow as shown by arrows in Fig. 3. Therefore, the stent 53 can be firmly expanded without obstructing the blood vessel 91 or causing disorder because of ischemia.

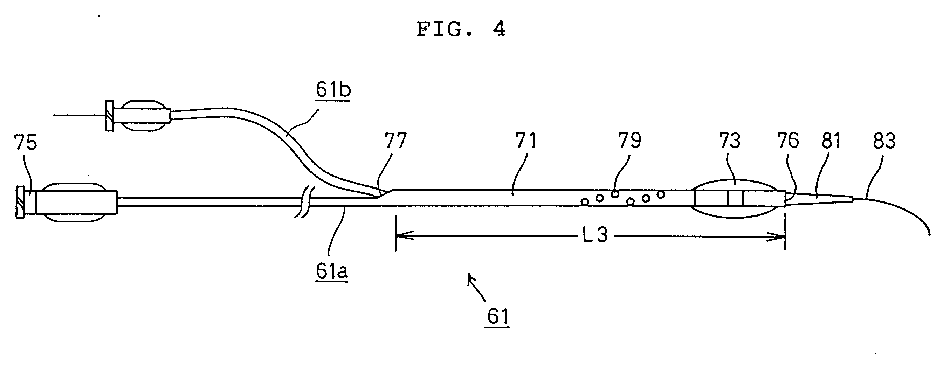

A second embodiment is now explained. While the perfusion catheter 1 of the first embodiment is of a so-called over-the-wire type, a perfusion catheter 61 of the second embodiment shown in Fig. 4 is of a monorail type or rapid exchange type.

Similar to the first embodiment, the perfusion catheter 61 is composed of an inner catheter 61b disposed inside an outer catheter 61a.

In the same manner as in the first embodiment, an outer tube 71 of outer catheter 61a is provided with first and second lumens inside. The first lumen leads to the inside of the balloon 73 at the forward end thereof and leads to a balloon expansion port 75 at the rear end thereof. Different from the first embodiment, the second lumen of the outer tube 71 is extended over a length L3. The second lumen leads from forward open end 76 of the balloon 73, preferably about 20cm to 30cm from the forward end of balloon 73, to a rear open end 77 at about the halfway position of the outer tube 71. The inner catheter 61b is slidably inserted through the rear open end 77 and a tapered tip 81 of the inner catheter 61b is projected from the forward open end 76. In the same manner as the first embodiment, side bores 79 formed in the side wall of the outer tube 71 communicate with the second lumen. When the tapered tip 81 of the inner catheter 61b is retracted past the side bores 79, the side bores 79 communicate via the second lumen to the forward open end 76, thereby permitting blood to flow. A lumen for passing through a guide wire 83 is formed in the inner catheter 61b.

In the same manner as in the first embodiment, the second lumen in the perfusion catheter 61 is enlarged for smoothly passing blood. Furthermore, the perfusion catheter 61 can be introduced in the blood vessel to the lesion portion via the tapered tip 81.

The advantages of the first embodiment of the perfusion catheter 1 are different than the advantages of the second embodiment of the perfusion catheter 61.

In the first embodiment of the perfusion catheter 1, the rear end of second lumen 23 leads to the inner catheter operation port 17. This configuration allows anticoagulant, or the like, to be injected via the inner catheter operation port 17 into the blood passage through the balloon 13. The introduction of the anticoagulant prevents the blood from clotting in the blood passage, so that the perfusion catheter 1 can be retained in the blood vessel for a long period of time.

In the second embodiment of the perfusion catheter 61, the outer catheter 61a and the inner catheter 61b are disposed parallel with each other behind the rear open end 77. This portion of the perfusion catheter 61, in which the outer and inner catheters 61a and 61b are disposed parallel, has a smaller cross-sectional area than a corresponding portion of the first embodiment of the perfusion catheter 1. Consequently, when the perfusion catheter 61 is introduced in the blood vessel via the guide catheter, greater clearance is provided between the outer wall of perfusion catheter 61 and the inner wall of the guide catheter. Therefore, an increased volume of contrast media can be injected through the clearance, so that the position of balloon 73 can be quickly and easily confirmed.

As aforementioned, the first embodiment provides different advantages than the second embodiment. For example, the first embodiment of the perfusion catheter 1 can be used for a long period of time, while the second embodiment of the perfusion catheter 61 can be used for a relatively short period of time.

According to the invention, the increased diameter of the blood passage facilitates blood flow. Furthermore, the movable tip member permits the advance of the perfusion catheter in the blood vessel and eases the penetration of the balloon 73 into the lesion portion of the blood vessel.

The over the wire type of the perfusion catheter is formed by inserting the guide wire along the axis of the perfusion catheter. This type of perfusion catheter can be easily guided in the blood vessel, and can be easily replaced with a new one.

In the present invention, the metallic wire 29 protects the blood passage in the perfusion catheter even when the balloon is swelled, thus applying high pressure to the inner wall of the perfusion catheter. The wire 29 ensures stable blood flow through the blood passage.

This invention has been described above with reference to the preferred embodiments as shown in the figures. Modifications and alterations may become apparent to one skilled in the art upon reading and understanding the specification. Despite the use of the embodiment for illustration purposes, the invention is intended to include all such modifications and alterations within the spirit and scope of the appended claims.

A perfusion catheter having an improved fluidity of blood in a blood passage and an improved operability in a blood vessel. The perfusion catheter is provided with an outer tube inserted through a balloon and an inner tube having a tapered or pointed tip disposed in the outer tube. The tapered tip of the inner tube is projected out of a forward open end of the outer tube and can retreat inside the forward open end. When the inner tube is moved back behind bores formed in a side wall of the outer tube, the bores communicate via a second lumen to the forward open end, thereby passing blood. The outer periphery inserted through the balloon of the outer tube is securely wound around with a coil.

## Claims

1. A perfusion catheter, comprising:
a catheter shaft having a proximal end, a distal end and a lumen for supplying and discharging a medium;
a balloon provided at said distal end of said catheter shaft, said balloon communicating with said lumen and having an adjustable size such that said balloon swells when said medium is supplied to said balloon, and said balloon contracts when said medium is discharged from said balloon;
a blood passage extending through said balloon for passing blood through said balloon; and
a tip member having a tapered end portion, said tip member being insertable into said blood passage provided in said balloon, such that, when said tip member is inserted into said blood passage, said tip member is movable between:
i) a forward position, in which said tip member occupies said blood passage and said tapered end portion protrudes from said blood passage, and
ii) a retracted position, in which said tip member is positioned to allow blood to flow through said blood passage.

2. A perfusion catheter according to claim 1, wherein said tapered end portion has a largest diameter about equal to a diameter of said blood passage provided in said balloon.

3. A perfusion catheter, comprising:
an outer cylindrical member having a proximal end, a distal end, a first lumen for supplying and discharging a medium, and a second lumen;
a balloon provided about said distal end of said outer cylindrical member, said balloon communicating with said first lumen and having an adjustable size such that, when said medium is supplied to said balloon, said balloon swells, and when said medium is discharged from said balloon, said balloon contracts;
a blood passage defined by:
i) bores extending through a sidewall of said outer cylindrical member into said second lumen, said bores located adjacent said balloon, towards said proximal end of said outer cylindrical member, and
ii) a portion of said second lumen extending from said bores, through said balloon and terminating in a forward open end; and
an inner cylindrical member having a tapered end portion, said inner cylindrical member being insertable into said second lumen, such that, when said inner cylindrical member is inserted into said second lumen, said inner cylindrical member is movable between:
i) a forward position, in which said inner cylindrical member occupies said blood passage and said tapered end portion protrudes from said forward open end, and
ii) a retracted position, in which said tapered end portion is located proximally adjacent said bores, such that at least one of said bores communicates through said second lumen with said forward open end.

4. A perfusion catheter according to claim 3, comprising a wire wound around said second lumen within said balloon.

5. A perfusion catheter according to claim 4, wherein said wire is helically wound around said second lumen.

6. A perfusion catheter according to claim 3, wherein said tapered end portion has a largest diameter about equal to a diameter of said forward end of said second lumen.

7. A perfusion catheter according to claim 3, wherein said inner cylindrical member is provided with a third lumen to accommodate one of a guide wire and a fluid.

8. A perfusion catheter according to claim 3, wherein said inner cylindrical member is an inner wire and said tapered end portion is secured to an end of said inner wire.

9. A perfusion catheter according to claim 3, wherein said second lumen extends through said balloon, such that said forward open end is distally spaced from said balloon by a predetermined distance.

10. A perfusion catheter according to claim 9, wherein said predetermined distance is in the range of about 20cm to about 30cm.

11. A perfusion catheter according to claim 3, wherein threads are provided on an outer surface of said inner cylindrical member, and said threads are engageable with corresponding threads provided on an inner surface of said second lumen, such that, when said threads are engaged, said inner cylindrical member is prevented from moving freely within said second lumen.
